# FASCICULE DE BREVET EUROPEEN

(11) **EP 2 613 792 B1**
(45) Date de publication et mention de la délivrance du brevet: **21.09.2016**
(21) Numéro de dépôt: 11773021.8
(22) Date de dépôt: 02.09.2011
(51) Int. Cl.: A61K 36/23, A61P 31/00

(54) **PROCÉDÉ DE PRÉPARATION D'UN EXTRAIT RAFFINÉ DE CENTELLA ASIATICA**
VERFAHREN FÜR DIE ZUBEREITUNG EINES RAFFINIERTEN EXTRAKTS AUS CENTELLA ASIATICA
METHOD FOR PREPARING A REFINED EXTRACT OF CENTELLA ASIATICA

(30) Priorité: 09.09.2010 FR 1057179
(43) Date de publication de la demande: 17.07.2013
(73) Titulaire: Naturex, 84140 Avignon (FR)
(72) Inventeur: DUVAL, Charles, F-71000 Macon (FR)
(74) Mandataire: Marek, Pierre
(86) Numéro de dépôt international: PCT/FR2011/052013
(87) Numéro de publication internationale: WO 2012/032250

(56) Documents cités:
- EP-A1- 2 030 626
- FR-A1- 2 915 899
- US-A1- 2008 194 499
- DATABASE WPI Week 200921 Thomson Scientific, London, GB; AN 2009-B49012 XP002628759, & CN 101 323 637 A (LU C) 17 décembre 2008 (2008-12-17)
- DATABASE WPI Week 200972 Thomson Scientific, London, GB; AN 2009-Q27498 XP002628760, & CN 101 549 075 A (SHANGHAI MODERN PHARMACY CO LTD) 7 octobre 2009 (2009-10-07)
- "Sepg. asiatic-oside from Centella asiatica - involves extracting dried plant with methanol , passing extract through anion-exchange resin column, concentration and crystallisation", DERWENT, 23 avril 1991 (1991-04-23), XP002251031,

## Description

La présente invention concerne un procédé de préparation d'un extrait raffiné de *Centella asiatica* comprenant un mélange de madécassoside, de terminoloside et d'asiaticoside.

*Centella asiatica* appartient à la famille des Ombellifères (Apiacées), particulièrement à la sous famille des Hydrocotyles. Cette plante est connue et utilisée par les médecines traditionnelles depuis plus de 3000 ans. Elle est particulièrement intéressante pour ses propriétés cicatrisantes, sédatives, analgésiques, antidépressives, anti-virales et anti-microbiennes.

Les composés actifs de *Centella asiatica* sont des triterpènes pentacycliques, qui se trouvent soit à l'état de triterpènes génines : acide asiatique (formule I) et acide madécassique (formule II), soit à l'état de triterpènes hétérosides : asiaticoside (formule III), madécassoside (formule IV) et terminoloside (formule V).

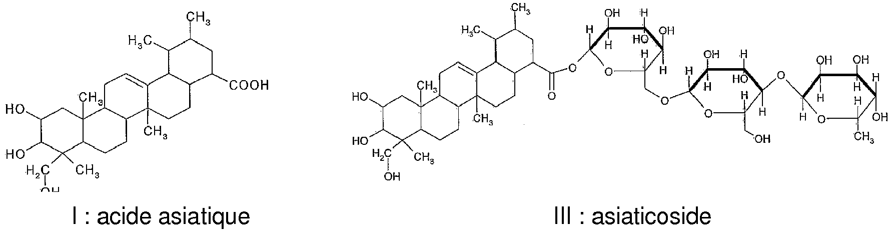

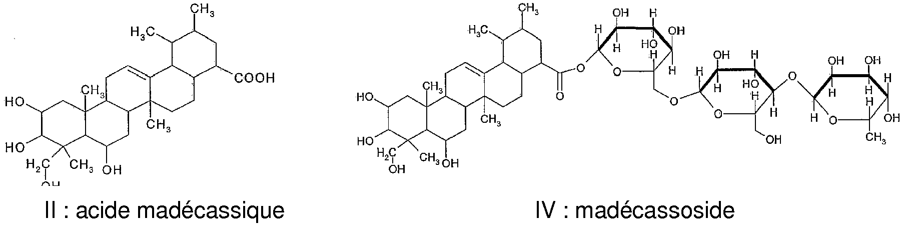

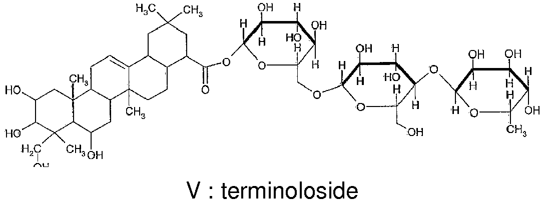

Le terminoloside est un isomère de position du madécassoside et présente le même enchaînement sucré, à savoir un enchaînement glucose-glucose-rhamnose. La structure du cycle terpénique du terminoloside correspond à celle du cycle terpénique de l'acide terminolique.

A la connaissance des inventeurs, aucun isomère de l'asiaticoside n'a été détecté à ce jour. On pourrait cependant supposer qu'il existe un isomère de position de l'asiaticoside, présentant une isomérie similaire à celle existant entre le madécassoside et le terminoloside.

Les hétérosides de *Centella asiatica,* asiaticoside et madécassoside, sont des complexes sucrés qui constituent respectivement les formes de réserve de l'acide asiatique et de l'acide madécassique de la plante.

Les molécules triterpéniques sont particulièrement intéressantes du fait de leur action régulatrice et activatrice de la synthèse de collagènes. Les génines et hétérosides extraits de *Centella asiatica* favorisent notamment la synthèse des collagènes 1 et 3. Ces actifs sont utilisés dans le domaine pharmaceutique principalement pour faciliter la cicatrisation et dans le traitement de l'insuffisance veineuse. Ils sont utilisés dans le domaine cosmétique principalement en tant qu'agent anti-rides et anti-cellulite. Les composés actifs de *Centella asiatica* les plus couramment utilisés dans l'art antérieur sont les acides asiatique et madécassique, ainsi que l'asiaticoside.

Le madécassoside étant très soluble dans l'eau, il est le plus souvent entraîné dans les eaux de lavage au cours des procédés d'extraction classiques des actifs liposolubles.

La demande internationale WO 2004/062678 propose un procédé de préparation d'un extrait de *Centella asiatica* comprenant un mélange de madécassoside, de terminoloside et d'asiaticoside, ledit procédé comprenant une étape de délipidation sélective par extraction liquide/liquide suivie d'une concentration de la phase hydro-alcoolique obtenue et de filtrations successives, cette séquence étant sensée retirer l'asiaticoside du mélange de madécassoside et de terminoloside. Cependant, le madécassoside solubilise une partie de l'asiaticoside, cette proportion résiduelle d'asiaticoside devant être éliminée par chromatographie sur gel de silice, technique de purification coûteuse et peu appréciée à l'échelle industrielle.

De manière surprenante, les inventeurs ont mis au point un nouveau procédé d'extraction qui permet la préparation d'un extrait raffiné de *Centella asiatica* comprenant un mélange de madécassoside, de terminoloside et d'asiaticoside. Dans ce procédé, les inventeurs ont mis en oeuvre une nouvelle étape, participant à la purification d'une solution comprenant un mélange de madécassoside, de terminoloside et d'asiaticoside.

La présente invention a pour objet l'utilisation d'une étape de passage sur résine adsorbante pour la préparation d'un extrait raffiné de *Centella asiatica* comprenant un mélange de madécassoside, de terminoloside et d'asiaticoside.

La résine adsorbante utilisée dans le procédé selon l'invention est avantageusement une résine propre à adsorber les triterpènes hétérosides contenus dans *Centella asiatica,* comme par exemple les résines adsorbantes de type styrène-divinylbenzène ou de type formophénolique.

Le procédé de préparation de l'extrait raffiné de *Centella asiatica* comprend les étapes suivantes :
α) le passage sur résine adsorbante d'une solution aqueuse comprenant un mélange de madécassoside, de terminoloside et d'asiaticoside, et
β) l'élution de ladite résine adsorbante par un éluant comprenant au moins 30% en masse d'un solvant alcoolique, et éventuellement de l'eau.

Dans le cadre de la présente description, on entend par « solvant alcoolique » un solvant comportant au moins une fonction alcool -OH, tel que le méthanol, l'éthanol, le propanol, le butanol, l'éthylène glycol, le glycérol, et plus particulièrement le méthanol et l'éthanol.

Lors de l'étape α), le passage sur résine adsorbante de la solution comprenant un mélange de madécassoside, de terminoloside et d'asiaticoside permet l'adsorption préférentielle de ces triterpènes hétérosides.

L'éluant utilisé lors de l'étape β) est éventuellement un solvant alcoolique pur ou avantageusement un mélange d'un solvant alcoolique et d'eau, comprenant de préférence de 50% à 80% de solvant alcoolique, préférentiellement de 60% à 70%.

Dans le procédé, l'étape α) est précédée de l'étape a) d'extraction des parties aériennes de *Centella asiatica* au moyen d'eau ou d'un mélange de solvants comprenant au moins 10% en masse d'eau, et éventuellement un solvant alcoolique,
ce par quoi on obtient une solution aqueuse comprenant un mélange de madécassoside, de terminoloside et d'asiaticoside.

Dans le cadre de la présente description, on entend par « parties aériennes de *Centella asiatica* » des feuilles de *Centella asiatica* constituées des limbes avec au maximum 5 cm de pétiole.

L'extraction des principes actifs de la plante de *Centella asiatica* est réalisée avantageusement à partir des parties aériennes de la plante, dans le but principal de ne pas détériorer ses racines et ainsi de permettre le renouvellement naturel de cette plante vivace.

Dans le procédé selon l'invention, l'étape d'extraction comprend une étape de macération pendant laquelle les parties aériennes sont mises à macérer sous agitation, dans de l'eau ou dans un mélange de solvants, comprenant de l'eau et éventuellement un solvant alcoolique.

Dans le cadre de la présente description, un « mélange de solvants » désigne particulièrement un mélange d'au moins deux solvants, de préférence deux solvants, comme par exemple de l'eau et un solvant alcoolique.

La macération est réalisée avantageusement au moyen d'eau ou d'un mélange de solvants comprenant au moins 10% en masse d'eau, et éventuellement un solvant alcoolique, à une température comprise de 20 à 90 °C, de préférence de 50 à 70°C, et préférentiellement à une température de l'ordre de 50°C, pendant une durée comprise de 20 à 180 minutes, dans un rapport [*Centella asiatica*]:[mélange de solvants] compris de 1:2 à 1:20.

Le mélange de macération obtenu est filtré pour fournir une solution aqueuse comprenant un mélange de madécassoside, de terminoloside et d'asiaticoside. Cette filtration est généralement opérée par filtrations successives, typiquement avec des filtres de porosité de 600 µm, 100 µm puis 25 µm.

Dans une variante du procédé selon l'invention, la solution aqueuse obtenue à l'issue de l'étape susmentionnée d'extraction est éventuellement concentrée, dans le but de diminuer les volumes à traiter et pour obtenir une solution concentrée comprenant de 2% à 25% de matière sèche.

La solution concentrée obtenue est éventuellement basifiée, avantageusement par addition d'une base forte minérale, comme par exemple un hydroxyde d'alcalin tel que la lithine LiOH, la soude NaOH ou la potasse KOH, sous forme solide ou d'une solution aqueuse, ou bien de la chaux vive CaO, ou encore de la chaux éteinte Ca(OH)₂, de manière à porter le pH de la solution concentrée à un pH de préférence compris de 8 à 12. La base forte minérale est de préférence choisie parmi NaOH, KOH et CaO.

L'ajout d'une base forte permet généralement la précipitation, et ainsi l'élimination par filtration, des métaux et des autres substances présentes susceptibles de réagir avec cette base forte. En général, les composés protéiques et résinoïdes précipités ou floculés sont ensuite éliminés du mélange basifié par filtration.

Le filtrat obtenu à l'issue de l'étape susmentionnée de filtration peut ensuite éventuellement être acidifié, avantageusement par addition d'un acide fort minéral, tel que l'acide phosphorique H₃PO₄, l'acide chlorhydrique HCl, l'acide nitrique HNO₃, l'acide sulfurique H₂SO₄, l'acide perchlorique HClO₄, l'acide bromhydrique HBr, ou bien d'un acide organique, comme par exemple un composé organique portant une ou plusieurs fonctions acide carboxylique -COOH, aromatique ou non-aromatique, tel que l'acide formique, l'acide éthanoïque, l'acide benzoïque, l'acide citrique ou l'acide carbonique. Cette étape d'acidification permet de porter le pH du filtrat à un pH de préférence compris entre 5 et 7. L'acide fort minéral est de préférence choisi parmi H₃PO₄ et HCl. L'acide organique est de préférence choisi parmi l'acide citrique ou l'acide carbonique.

Les sels formés et les composés précipités sont généralement éliminés du mélange acidifié par filtration.

Dans le procédé, l'étape β) est suivie des étapes suivantes :
b) le passage sur résine décolorante de l'éluat obtenu à l'issue de l'étape β), ce par quoi on obtient une phase aqueuse décolorée, et
c) la concentration sous vide de la phase aqueuse décolorée,
ce par quoi on obtient un extrait de *Centella asiatica* comprenant un mélange de madécassoside, de terminoloside et d'asiaticoside.

A ce point, le mélange obtenu est constitué de madécassoside, de 25% à 65% de terminoloside et de 20% à 40% d'asiaticoside.

De préférence, l'extrait ainsi obtenu comprend de 25% à 65% en masse d'un mélange de terminoloside et de madécassoside, et de 20% à 40% en masse d'asiaticoside, par rapport à la masse totale de l'extrait.

Dans le procédé selon l'invention, l'éluat obtenu à l'issue de l'étape β) est d'abord traité par du charbon activé. L'ajout de charbon activé permet généralement de décolorer l'éluat, en fixant des acides gras ou des produits d'oxydation.

Cet éluat, traité par du charbon activé, peut ensuite être concentré par distillation du ou des solvant(s) alcoolique(s). L'éluat concentré est ensuite filtré pour permettre l'élimination des composés insolubles dans la phase aqueuse. Une partie de l'asiaticoside précipite généralement lors de la concentration de l'éluat et est éliminé par filtration.

Lors de l'étape b), l'éluat obtenu à l'issue de l'étape β), traité par du charbon activé, concentré et filtré, est décoloré par passage sur résine décolorante, par exemple une résine de type anionique forte styrène-chlorure.

Dans le procédé selon l'invention, la phase aqueuse décolorée est ensuite traitée par passage successif sur une résine échangeuse d'ions cationique puis sur une résine échangeuse d'ions anionique, de manière à retirer les sels dissous et les composés indésirables de la phase aqueuse. La résine cationique utilisée est une résine cationique forte possédant des groupements fonctionnels du type sulfonates. La résine anionique utilisée est une résine anionique forte possédant des groupements fonctionnels du type ammonium quaternaire. Dans le cadre du procédé selon l'invention, l'ordre des résines échangeuses d'ions est important afin d'améliorer la fixation de fractions acides résiduelles. L'ordre peut néanmoins être interverti au besoin.

Le passage successif sur résines cationique et anionique peut être avantageusement répété, voire même réalisé par un passage de la phase aqueuse décolorée sur un lit mélangé desdites résines.

Lors de l'étape c), la phase aqueuse décolorée, traitée par passage sur résines cationique et anionique, est concentrée sous vide, puis le concentré obtenu est généralement séché sous vide ou par atomisation. La concentration est réalisée par des techniques membranaires de filtration tangentielle sur membrane organique ou minérale, par l'usage de media ayant un seuil de coupure compris de 200 Da à 100 kDa (c'est-à-dire par l'utilisation de molécules ayant un poids moléculaire compris entre 200 et 100 000 g/mol).

A l'issue de l'étape c), on obtient un extrait de *Centella asiatica* comprenant généralement de 25 à 65% en masse d'un mélange de madécassoside et de terminoloside, et de 20 à 40% en masse d'asiaticoside, par rapport à la masse totale de l'extrait.

A l'issue de l'étape c), on obtient un extrait de *Centella asiatica* comprenant généralement de 25 à 65% en masse d'un mélange de madécassoside et de terminoloside par rapport à la masse totale de l'extrait.

A l'issue de l'étape c), on obtient un extrait de *Centella asiatica* comprenant généralement de 20 à 40% en masse d'asiaticoside, par rapport à la masse totale de l'extrait.

Dans le cadre de la présente invention, on entend par le terme « asiaticoside » la molécule chimique de formule III, éventuellement en mélange avec un de ses isomères oléaniques.

La présente invention a pour objet un procédé de préparation d'un extrait raffiné de *Centella asiatica* comprenant un mélange de madécassoside, de terminoloside et d'asiaticoside, ledit procédé comprenant une étape de recristallisation de l'extrait obtenu selon le procédé précédemment décrit, au moyen d'un mélange de solvants comprenant de l'eau et un solvant alcoolique.

A titre de solvant alcoolique adapté à l'étape de recristallisation, on peut citer par exemple l'éthanol, le méthanol et le butanol, ou un mélange de ceux-ci.

Dans le cadre de la présente description, on entend par « extrait raffiné de *Centella asiatica* » un extrait de *Centella asiatica* comprenant plus de 50% en masse d'un mélange de madécassoside et de terminoloside, par rapport à la masse totale de l'extrait.

Un tel extrait comprend moins de 15% en masse d'asiaticoside par rapport à la masse totale de l'extrait.

L'étape de recristallisation du procédé selon l'invention permet de retirer la majeure partie de l'asiaticoside et ainsi d'augmenter la teneur du mélange de madécassoside et terminoloside dans l'extrait raffiné obtenu.

La technique de purification par recristallisation est fondée sur la différence de solubilité à chaud et à froid de composés dans des solvants. La solubilité d'un composé solide augmente généralement avec la température. Aussi, lorsqu'il est solubilisé dans un solvant chaud, sa cristallisation peut-être provoquée par le refroidissement de la solution jusqu'à sa sursaturation. La recristallisation consiste donc en la mise en solution du composé solide à purifier dans un solvant ou dans un mélange de solvants, généralement à l'ébullition, puis au refroidissement de la solution, ce qui entraîne la cristallisation du solide, isolé ensuite par filtration. On peut également concentrer le soluté jusqu'à précipitation par évaporation du solvant.

Plus précisément, l'étape de recristallisation comprend les étapes suivantes :
d) la solubilisation de l'extrait obtenu à l'issue de l'étape c) selon le procédé décrit précédemment, dans un mélange de solvants comprenant de l'eau et un solvant alcoolique, porté à une température comprise de 40 °C à 70 °C, ce par quoi on obtient une solution,
e) la diminution de la température de la solution obtenue à l'issue de l'étape d) vers une température comprise de -20°C à 30°C, ce par quoi on obtient un mélange refroidi,
f) la filtration du mélange refroidi, ce par quoi on obtient un filtrat,
g) la concentration du filtrat,

ce par quoi on obtient un extrait raffiné de *Centella asiatica* comprenant un mélange de madécassoside, de terminoloside et d'asiaticoside.

Dans le cadre de l'invention, le mélange de solvants de recristallisation est un mélange de solvants comprenant de l'eau et un solvant alcoolique. Un mélange de solvants de recristallisation particulièrement avantageux comprend 70% d'eau et 30% d'un solvant alcoolique, de préférence l'éthanol ou le méthanol, de préférence porté à une température comprise de 55°C à 65°C, préférentiellement de l'ordre de 60°C.

Le mélange de solvants de recristallisation est éventuellement porté à ébullition pour permettre la solubilisation de l'extrait à purifier.

Lors de la solubilisation de l'extrait obtenu à l'issue de l'étape c), le mélange de solvants est généralement additionné au goutte à goutte, de sorte que l'on puisse arrêter l'addition de solvant lorsque la totalité de l'extrait est solubilisé.

La solution obtenue est ensuite refroidie de manière à provoquer la cristallisation de l'asiaticoside. La diminution de température est généralement lente et graduelle, sur une période de temps comprise de 1 à 250 heures, de préférence de 100 à 250 heures, de manière à augmenter la pureté des cristaux d'asiaticoside formés et ainsi ne pas emprisonner de terminoloside et de madécassoside dans ces cristaux, ce qui aurait pour effet de faire baisser la teneur en ces composés dans les eaux mères. La température du mélange refroidi est généralement comprise entre -10°C et 10°C, de préférence entre -5°C et 5°C.

Le mélange refroidi, comprenant les cristaux formés et les eaux mères comprenant notamment le terminoloside et le madécassoside, est filtré pour retirer les composés solides cristallisés et/ou précipités insolubles à la température atteinte. Les cristaux ainsi écartés contiennent généralement plus de 75% en asiaticoside et peuvent faire l'objet d'une valorisation.

Le filtrat - encore appelé eaux mères - est concentré par distillation, puis généralement séché sous vide.

On obtient un extrait raffiné de *Centella asiatica* comprenant plus de 50% en masse d'un mélange de madécassoside et de terminoloside, voire plus de 60%, voire plus de 70%, par rapport à la masse totale de l'extrait.

Cet extrait comprend par ailleurs moins de 15% en masse d'asiaticoside, voire moins de 10% par rapport à la masse totale de l'extrait.

L'invention propose ainsi un procédé de préparation d'un extrait raffiné de *Centella asiatica* comprenant plus de 50% en masse d'un mélange de madécassoside et de terminoloside par rapport à la masse totale de l'extrait, conforme à la revendication 1. Pour plus de clarté, les étapes dudit procédé sont reprises ci-après :
a. Macération sous agitation des parties aériennes ou feuilles de la plante *Centella asiatica* dans de l'eau ou dans un mélange de solvants comprenant au moins de l'eau et un solvant alcoolique, à une température de 20 à 90°C, pendant 20 à 180 minutes, dans un rapport [*Centella asiatica*]:[mélange de solvants] compris entre 1:2 à 1:20, puis :
   filtration du mélange de macération, ce par quoi on obtient une solution aqueuse
α. Passage de la solution aqueuse obtenue sur une résine adsorbante propre à adsorber les triterpènes hétérosides
β. Elution de ladite résine adsorbante par un éluant comprenant au moins 30% en masse d'un solvant alcoolique et éventuellement de l'eau, ce par quoi on obtient un éluat, puis :
   traitement de l'éluat obtenu par du charbon activé
b. Passage de l'éluat traité sur une résine décolorante de type anionique forte styrène-chlorure puis traitement par passage(s) successif(s) sur une résine échangeuse d'ions cationique forte comprenant des groupements fonctionnels du type sulfonates, puis sur une résine d'ions anionique forte comprenant des groupements fonctionnels du type ammonium quaternaire, ou passage de l'éluat sur lesdites résines mélangées, ce par quoi on obtient une phase aqueuse décolorée
c. Concentration sous vide de la phase aqueuse décolorée par des techniques membranaires de filtration tangentielle sur membrane organique ou minérale par l'usage de média ayant un seuil de coupure compris de 200Da à 100kDA, ce par quoi on obtient un extrait, puis

Recristallisation de l'extrait obtenu par :
d. Solubilisation de l'extrait obtenu dans un mélange de solvants comprenant de l'eau et un solvant alcoolique, porté à une température de 40°c à 70°c, ce par quoi on obtient une solution
e. Diminution lente et graduelle de la température pendant 1 à 250 heures vers une température du mélange refroidi comprise entre -20°c et 30°c, ce par quoi on obtient un mélange refroidi
f. Filtration du mélange refroidi, ce par quoi on obtient un filtrat
g. Concentration par distillation du filtrat, ce par quoi on obtient un extrait raffiné comprenant moins de 15% en masse d'asiaticoside par rapport à la masse totale de l'extrait

La présente invention a également pour objet un procédé de préparation d'un extrait purifié de *Centella asiatica* comprenant un mélange de madécassoside et de terminoloside, ledit procédé comprenant une étape de purification de l'extrait raffiné obtenu à l'issue de l'étape g) selon le procédé précédemment décrit, au moyen d'un mélange de solvants comprenant de l'eau et un solvant alcoolique.

Dans le cadre de la présente description, on entend par « extrait purifié de *Centella asiatica* » un extrait de *Centella asiatica* comprenant plus de 90% en masse d'un mélange de madécassoside et de terminoloside, par rapport à la masse totale de l'extrait. Un tel extrait contient généralement moins de 6% en masse d'asiaticoside, par rapport à la masse totale de l'extrait.

L'étape de purification du procédé selon l'invention permet généralement de retirer plus de 40%, voire plus de 50% de la proportion d'asiaticoside présente dans un extrait raffiné de *Centella asiatica.*

Selon une première variante du procédé de l'invention, l'étape de purification comprend les étapes suivantes : la masse totale de l'extrait.

L'étape de purification du procédé selon l'invention permet généralement de retirer plus de 40%, voire plus de 50% de la proportion d'asiaticoside présente dans un extrait raffiné de *Centella asiatica.*

Selon une première variante du procédé de l'invention, l'étape de purification comprend les étapes suivantes:
h) l'addition à l'extrait raffiné obtenu à l'issue de l'étape g) selon le procédé précédemment décrit, d'un mélange de solvants comprenant un solvant alcoolique, et éventuellement de l'eau, porté à une température comprise de 25°C à 65°C, ce par quoi on obtient une solution,
i) la diminution de la température de la solution vers une température comprise de -20°C à 30°C, ce par quoi on obtient un mélange refroidi,
j) la filtration du mélange refroidi, ce par quoi on obtient un filtrat,
k) la concentration du filtrat,

ce par quoi on obtient un extrait purifié de *Centella asiatica* comprenant un mélange de madécassoside et de terminoloside.

Le mélange de solvants additionné lors de l'étape h), à un extrait raffiné obtenu à l'issue de l'étape g) selon le procédé précédemment décrit, comprend avantageusement au moins 85% en masse du solvant alcoolique et de préférence au moins 95%. Ce mélange de solvants est avantageusement porté à une température comprise de 30°C à 50°C, préférentiellement à une température de l'ordre de 45°C.

Lors de la solubilisation de l'extrait obtenu à l'issue de l'étape g), le mélange de solvants est généralement additionné au goutte à goutte, de sorte que l'on puisse arrêter l'addition de solvant lorsque la totalité de l'extrait est solubilisé.

La solution obtenue est ensuite refroidie, généralement de manière lente et graduelle, de préférence sur une période de temps comprise entre 1 et 250 heures, de préférence entre 100 et 250 heures, de préférence jusqu'à une température comprise entre -15°C et 15°C, avantageusement comprise entre -10°C et 0°C, et préférentiellement autour de -5°C.

Le mélange refroidi, comprenant les cristaux formés et les eaux mères comprenant notamment le terminoloside et le madécassoside, est filtré pour retirer les composés solides cristallisés et/ou précipités insolubles à la température atteinte. Le filtrat - encore appelé eaux mères - est concentré par distillation, puis séché sous vide.

A l'issue de l'étape de purification du procédé de l'invention, on obtient un extrait purifié de *Centella asiatica* comprenant généralement plus de 90% en masse d'un mélange de madécassoside et de terminoloside, par rapport à la masse totale de l'extrait. Cet extrait comprend par ailleurs moins de 6% en masse d'asiaticoside par rapport à la masse totale de l'extrait.

Cette étape de purification peut être avantageusement répétée deux à trois fois afin d'augmenter la teneur en madécassoside et de terminoloside de l'extrait purifié de *Centella asiatica.* La répétition de cette étape de purification permet d'atteindre plus de 95%, voire plus de 98% en masse d'un mélange de madécassoside et de terminoloside, par rapport à la masse totale de l'extrait, et moins de 3%, voire moins de 1% en masse d'asiaticoside par rapport à la masse totale de l'extrait.

L'extrait raffiné de *Centella asiatica* obtenu à l'issue de l'étape g) et l'extrait purifié de *Centella asiatica* obtenu à l'issue de l'étape k) peuvent éventuellement faire l'objet d'une purification ultérieure par chromatographie préparative sur gel de silice afin d'augmenter leur teneur respective en madécassoside et terminoloside. Cette étape de chromatographie est effectuée en utilisant, comme solvant d'élution, un mélange de solvants comprenant un solvant alcoolique et de l'eau.

Dans le cadre de la présente description, l'éluant utilisé lors de la chromatographie préparative est généralement un solvant polaire. Avantageusement, le solvant est un mélange d'eau et d'un solvant alcoolique, tel que l'éthanol, comprenant de préférence de l'eau et de l'éthanol dans les proportions 1/1 (50% d'eau et 50% d'éthanol).

Dans le cadre de la présente invention, la phase stationnaire utilisée lors de la chromatographie préparative est une phase stationnaire apolaire. Avantageusement, la phase stationnaire est constituée de silices apolaires greffées. Les greffons apolaires ont avantageusement de 2 à 18 atomes de carbone, et encore plus avantageusement de 12 à 18 atomes de carbone.

La présente invention a également pour objet un procédé de préparation d'un extrait purifié de *Centella asiatica* comprenant un mélange de madécassoside et de terminoloside, ledit procédé comprenant les étapes suivantes :
a) l'extraction des parties aériennes de *Centella asiatica* au moyen d'eau ou d'un mélange de solvants comprenant au moins 10% en masse d'eau, et éventuellement un solvant alcoolique, ce par quoi on obtient une solution,
α) le passage sur résine adsorbante de la solution,
β) l'élution de ladite résine adsorbante par un éluant comprenant au moins 30% en masse d'un solvant alcoolique, et éventuellement de l'eau, ce par quoi on obtient un éluat,
b) le passage sur une résine décolorante de l'éluat, ce par quoi on obtient une phase aqueuse décolorée,
c) la concentration de la phase aqueuse décolorée, ce par quoi on obtient un extrait,
d) la solubilisation de l'extrait obtenu à l'issue de l'étape c), dans un mélange de solvants comprenant de l'eau et un solvant alcoolique, porté à une température comprise de 40°C à 70°C, ce par quoi on obtient une solution,
e) la diminution de la température de la solution obtenue à l'issue de l'étape d) vers une température comprise de -20°C à 30°C, ce par quoi on obtient un mélange refroidi,
f) la filtration du mélange refroidi, ce par quoi on obtient un filtrat,
g) la concentration du filtrat, ce par quoi on obtient un extrait raffiné,
h) l'addition à l'extrait raffiné obtenu à l'issue de l'étape g) d'un mélange de solvants comprenant un solvant alcoolique, et éventuellement de l'eau, porté à une température comprise de 25°C à 65°C, ce par quoi on obtient une solution,
i) la diminution de la température de la solution vers une température comprise de -20°C à 30°C, ce par quoi on obtient un mélange refroidi,
j) la filtration du mélange refroidi, ce par quoi on obtient un filtrat, et
k) la concentration du filtrat,
ce par quoi on obtient un extrait purifié de *Centella asiatica* comprenant un mélange de madécassoside et de terminoloside.

### EXEMPLES

### Exemple 1

### • Préparation d'un extrait brut de Centella asiatica

Un kilo de feuilles de *Centella asiatica* (feuilles coupées) est plongé dans 20 litres d'eau portés à 70°C. Le mélange est laissé à macérer sous agitation pendant 2 heures, puis est filtré par filtrations successives sur des filtres de porosité croissante (600 µm, puis 100 µm, puis 25 µm).

On obtient 16,2 litres d'un extrait brut sous forme liquide, comprenant 2% de matière sèche, avec un titre de 11,8% en masse d'un mélange de madécassoside et de terminoloside, et un titre de 10,9% en masse d'asiaticoside, par rapport à la masse totale de matière sèche.

### • Préparation d'un extrait semi-raffiné de Centella asiatica

L'extrait brut sous forme liquide est passé sur résine adsorbante de type RES-00820 (Burgundy), puis ladite résine est éluée par un éluant constitué de 70% d'éthanol et 30% d'eau vol/vol.

L'éluat éthanolique récupéré est ensuite passé sur résine décolorante anionique de type RES-01541 (Burgundy), puis la phase décolorée est désalcoolisée par distillation et concentrée sous vide, ce par quoi on obtient un concentré aqueux décoloré. Ce concentré est ensuite séché par lyophilisation.

On obtient 70 g d'un extrait semi-raffiné, avec un titre de 47,2% en masse d'un mélange de madécassoside et de terminoloside, et un titre de 37,4% en masse d'asiaticoside, par rapport à la masse totale de l'extrait.

### • Préparation de l'extrait raffiné de Centella asiatica

L'extrait semi-raffiné est solubilisé dans une solution comprenant 70% d'eau et 30% de méthanol, portée à une température de 60°C. Après solubilisation totale de l'extrait, la température de la solution est diminuée sur une période de 240 h jusqu'à une température de 5°C (cryostat puis stockage en chambre froide).

Le mélange refroidi obtenu est filtré, puis le filtrat est désalcoolisé par distillation puis concentré sous vide, ce par quoi on obtient un concentré aqueux raffiné. Ce concentré est ensuite séché par lyophilisation.

On obtient 45 g d'un extrait raffiné, avec un titre de 67,2% en masse d'un mélange de madécassoside et de terminoloside, et un titre de moins de 10% (4,5%) en masse d'asiaticoside, par rapport à la masse totale de l'extrait.

### • Préparation d'un extrait purifié de Centella asiatica

L'extrait raffiné est solubilisé dans une solution comprenant 5% d'eau et 95% de méthanol, portée à une température de 45°C. Après solubilisation totale de l'extrait, la température de la solution est diminuée sur une période de 120 h jusqu'à une température de 5°C.

Le mélange refroidi obtenu est filtré, puis le filtrat est désalcoolisé par distillation puis concentré sous vide, ce par quoi on obtient un concentré aqueux purifié. Ce concentré est ensuite séché par lyophilisation.

On obtient 20 g d'un extrait purifié, titrant plus de 90% (91,2%) en masse d'un mélange de madécassoside et de terminoloside, et titrant moins de 6% (3,1%) en masse d'asiaticoside, par rapport à la masse totale de l'extrait.

### Exemple 2

### • Préparation d'un extrait brut de Centella asiatica

Un kilo de feuilles de *Centella asiatica* (feuilles coupées) est plongé dans 15 litres d'éthanol 70% vol. portés à 50°C. Le mélange est laissé à macérer sous agitation pendant 2 heures, puis est filtré par filtrations successives sur des filtres de porosité croissante (600 µm, puis 100 µm, puis 25 µm).

Le filtrat est ensuite désalcoolisé par distillation, puis le filtrat concentré est filtré.

On obtient 3,7 litres d'un extrait brut sous forme liquide, comprenant 9% de matière sèche, avec un titre de 11,2% en masse d'un mélange de madécassoside et de terminoloside, et un titre de 8,6% en masse d'asiaticoside, par rapport à la masse totale de matière sèche.

### • Préparation d'un extrait semi-raffiné de Centella asiatica

L'extrait brut sous forme liquide est passé sur résine adsorbante de type RES-00408 (Burgundy), puis ladite résine est éluée par un éluant constitué de 60% d'éthanol et 40% d'eau vol/vol.

L'éluat éthanolique récupéré est ensuite désalcoolisé par distillation puis est passé sur résine décolorante anionique de type RES-01953 (Burgundy), puis sur résine échangeuse d'ions de type cationique RES-01747 (Burgundy), puis sur une résine échangeuse d'ions de type anionique RES-02056 (Burgundy). La phase décolorée et déminéralisée obtenue est concentrée sous vide, ce par quoi on obtient un concentré aqueux décoloré et déminéralisé. Ce concentré est ensuite filtré puis séché par lyophilisation (éventuellement remplacée par une atomisation).

On obtient 60 g d'un extrait semi-raffiné, avec un titre de 47,2% en masse d'un mélange de madécassoside et de terminoloside, et un titre de 27,4% en masse d'asiaticoside, par rapport à la masse totale de l'extrait.

### • Préparation d'un extrait raffiné de Centella asiatica

L'extrait semi-raffiné est solubilisé dans une solution comprenant 70% d'eau et 30% de méthanol, portée à une température de 60°C. Après solubilisation totale de l'extrait, la température de la solution est diminuée sur une période de 120 h jusqu'à une température de -5°C (cryostat puis stockage en chambre froide).

Le mélange refroidi obtenu est filtré, puis le filtrat est désalcoolisé par distillation puis concentré sous vide, ce par quoi on obtient un concentré aqueux raffiné. Ce concentré est ensuite séché par lyophilisation.

On obtient 35 g d'un extrait raffiné, avec un titre de 67,5% en masse d'un mélange de madécassoside et de terminoloside, et un titre de 13,2% en masse d'asiaticoside, par rapport à la masse totale de l'extrait.

### • Préparation d'un extrait purifié de Centella asiatica

L'extrait raffiné est solubilisé dans une solution comprenant 5% d'eau et 95% de méthanol, portée à une température de 45°C. Après solubilisation totale de l'extrait, la température de la solution est diminuée sur une période de 120 h jusqu'à une température de 5°C.

Le mélange refroidi obtenu est filtré, puis le filtrat est désalcoolisé par distillation puis concentré sous vide, ce par quoi on obtient un concentré aqueux purifié. Ce concentré est ensuite séché par lyophilisation.

On obtient 20 g d'un extrait purifié, titrant plus de 90% (91,5%) en masse d'un mélange de madécassoside et de terminoloside, et titrant moins de 6% (4,6%) en masse d'asiaticoside, par rapport à la masse totale de l'extrait.

## Revendications

1. Procédé de préparation d'un extrait raffiné de *Centella asiatica* comprenant plus de 50% en masse d'un mélange de madécassoside et de terminoloside par rapport à la masse totale de l'extrait, ledit procédé comprenant les étapes suivantes :
a. Macération sous agitation des parties aériennes ou feuilles de la plante *Centella asiatica* dans de l'eau ou dans un mélange de solvants comprenant au moins de l'eau et un solvant alcoolique, à une température de 20 à 90°C, pendant 20 à 180 minutes, dans un rapport [*Centella asiatica*] :[mélange de solvants] compris entre 1:2 à 1:20, puis :
filtration du mélange de macération, ce par quoi on obtient une solution aqueuse
α. Passage de la solution aqueuse obtenue sur une résine adsorbante propre à adsorber les triterpènes hétérosides
β. Elution de ladite résine adsorbante par un éluant comprenant au moins 30% en masse d'un solvant alcoolique et éventuellement de l'eau, ce par quoi on obtient un éluat, puis :
traitement de l'éluat obtenu par du charbon activé
b. Passage de l'éluat traité sur une résine décolorante de type anionique forte styrène-chlorure puis traitement par passage(s) successif(s) sur une résine échangeuse d'ions cationique forte comprenant des groupements fonctionnels du type sulfonates, puis sur une résine d'ions anionique forte comprenant des groupements fonctionnels du type ammonium quaternaire, ou passage de l'éluat sur lesdites résines mélangées, ce par quoi on obtient une phase aqueuse décolorée
c. Concentration sous vide de la phase aqueuse décolorée par des techniques membranaires de filtration tangentielle sur membrane organique ou minérale par l'usage de média ayant un seuil de coupure compris de 200Da à 100kDA, ce par quoi on obtient un extrait, puis
Recristallisation de l'extrait obtenu par :
d. Solubilisation de l'extrait obtenu dans un mélange de solvants comprenant de l'eau et un solvant alcoolique, porté à une température de 40°c à 70°c, ce par quoi on obtient une solution
e. Diminution lente et graduelle de la température pendant 1 à 250 heures vers une température du mélange refroidi comprise entre -20°c et 30°c, ce par quoi on obtient un mélange refroidi
f. Filtration du mélange refroidi, ce par quoi on obtient un filtrat
g. Concentration par distillation du filtrat, ce par quoi on obtient un extrait raffiné comprenant moins de 15% en masse d'asiaticoside par rapport à la masse totale de l'extrait

2. Procédé selon la revendication 1 dans lequel le mélange de solvants dans l'étape a) est un mélange d'au moins deux solvants comprenant au moins 10% en masse d'eau et un solvant alcoolique

3. Procédé selon la revendication 1 ou 2 dans lequel la macération des parties aériennes de *Centella asiatica* est effectuée à une température comprise entre 50°C et 70°C

4. Procédé selon l'une quelconque des revendications 1 à 3 dans lequel la macération des parties aériennes de *Centella asiatica* est effectuée à une température de l'ordre de 50°C

5. Procédé selon l'une quelconque des revendications 1 à 4 dans lequel la filtration du mélange de macération est effectué par filtrations successives avec des filtres de porosité de 600µm, 100µm puis 25µm

6. Procédé selon l'une quelconque des revendications 1 à 5 dans lequel l'étape α) est précédée des étapes suivantes :
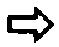 concentration du filtrat obtenu à l'issue de l'étape a) puis
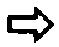 basification avec une base forte minérale de type hydroxyde alcalin (lithine LiOH, soude NaOH ou potasse KOH) sous forme solide ou de solution aqueuse, ou de la chaux vive CaO, ou encore de la chaux éteinte Ca(OH)₂, puis 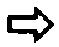
acidification avec un acide fort minéral de type acide phosphorique H₃PO₄ ,acide phosphorique HCl, acide nitrique HNO₃, acide sulfurique H₂SO₄, acide perchlorique HClO₄, acide bromhydrique Hbr, ou bien acide organique
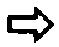 filtration du mélange acidifié

7. Procédé selon l'une quelconque des revendications 1 à 6 **caractérisé en ce que** la résine adsorbante de l'étape α est de type styrène-divinylbenzene ou de type formophénolique

8. Procédé selon l'une quelconque des revendications 1 à 7 **caractérisé en ce que** l'éluant dans l'étape β comprend 60 à 70% en masse d'un solvant alcoolique et plus particulièrement 60 à 70% d'éthanol, et de l'eau

9. Procédé selon l'une quelconque des revendications 1 à 8 dans lequel l'étape b) est précédée des étapes suivantes :
- concentration de l'éluat obtenu par distillation du ou des solvants alcooliques
- filtration de l'éluat distillé

10. Procédé selon l'une quelconque des revendications 1 à 9 dans lequel l'étape d) est précédée d'une étape de séchage sous vide ou par atomisation

11. Procédé selon l'une quelconque des revendications 1 à 10 **caractérisé en ce que** le mélange de solvants additionné à l'étape d) comprend 70% d'eau et 30% de méthanol et est porté à une température de l'ordre de 60°C

12. Procédé selon l'une quelconque des revendications 1 à 11 **caractérisé en ce que** la diminution de la température à l'étape e) s'effectue pendant une durée de 100 à 250 heures, vers une température du mélange refroidi comprise entre -5°C et 5°C

13. Procédé de préparation d'un extrait purifié de *Centella asiatica* comprenant plus de 90% en masse d'un mélange de madécassoside et de terminoloside par rapport à la masse totale de l'extrait, ledit procédé comprenant une étape de purification de l'extrait raffiné obtenu selon le procédé selon l'une quelconque des revendications 1 à 12, ladite étape de purification comprenant les étapes suivantes :
h. Addition à l'extrait raffiné obtenu selon le procédé selon l'une quelconque des revendications 1 à 12 d'un mélange de solvants comprenant au moins 85% en masse d'un solvant alcoolique et éventuellement de l'eau, porté à une température comprise entre 30°c et 50°c, ce par quoi on obtient une solution
i. Diminution lente et graduelle de la température de la solution pendant 1 à 250 heures vers une température comprise entre -15°c à 15°c, ce par quoi on obtient un mélange refroidi
j. Filtration du mélange refroidi ce par quoi on obtient un filtrat
k. Concentration par distillation du filtrat, ce par quoi on obtient un extrait purifié comprenant moins de 6%, voire moins de 1 % en masse d'asiaticoside par rapport à la masse totale de l'extrait

14. Procédé selon la revendication 13 **caractérisé en ce que** le mélange de solvants à l'étape h) est porté à une température de l'ordre de 45°C

15. Procédé selon la revendication 13 ou 14 **caractérisé en ce que** la diminution de la température à l'étape i) s'effectue pendant une durée de 100 à 250 heures, vers une température du mélange refroidi autour de -5°C

## Patentansprüche

1. Verfahren für die Zubereitung eines raffinierten Extrakts aus *Centella asiatica* mit über 50 % Massenanteil einer Mischung aus Madecassoside und Terminoloside gegenüber der Gesamtmasse des Extrakts, wobei das Verfahren die folgenden Schritte umfasst:
a. Mazeration der überirdischen Teile oder Blätter der Pflanze Centella *asiatica* unter Schütteln in Wasser oder in einer Mischung aus Lösungsmitteln, die zumindest Wasser und ein alkoholisches Lösungsmittel umfasst, bei einer Temperatur von 20 bis 90 °C während 20 bis 180 Minuten in einem Verhältnis [*Centella asiatica*] : [Lösungsmittelgemisch] zwischen 1:2 und 1:20, und anschließend:
Filtration des Mazerationsgemischs, wodurch man eine wässrige Lösung erhält
α Aufgabe der erhaltenen wässrigen Lösung auf ein Adsorberharz, das geeignet ist, die Triterpene-Heteroside zu adsorbieren
β Elution des Adsorberharzes mit einem Eluenten, der zumindest 30 % Massenanteil eines alkoholischen Lösungsmittels und gegebenenfalls Wasser enthält, wodurch man ein Eluat erhält, und anschließend:
Aufbereitung des erhaltenen Eluats mittels Aktivkohle
b. Aufgabe des aufbereiteten Eluats auf ein entfärbendes Harz vom Typ stark anionisches Styrol-Chlorid und anschließende Aufbereitung durch aufeinanderfolgende Aufgabe(n) auf ein stark kationisches Ionenaustauscherharz, das Funktionsgruppen vom Typ Sulfonate enthält, und dann auf ein stark anionisches Ionenaustauscherharz, das Funktionsgruppen vom Typ quaternäres Ammonium enthält, oder Aufgabe des Eluats auf eine Mischung der Harze, wodurch man eine entfärbte wässrige Phase enthält
c. Konzentration der entfärbten wässrigen Phase unter Vakuum mittels Kreuzstrom-Membranfiltertechniken auf einer organischen oder mineralischen Membran durch den Einsatz von Medien mit einer Ausschlusegrenze zwischen 200 Da und 100 kDA, wodurch man ein Extrakt erhält, und anschließend Rekristallisation des erhaltenen Extrakts durch:
d. Auflösung des erhaltenden Extrakts in einer Lösungsmittelmischung, die Wasser und ein alkoholisches Lösungsmittel enthält und die auf eine Temperatur zwischen 40 °C und 70 °C erhitzt wird, wodurch man eine Lösung erhält
e. Langsames und schrittweises Absenken der Temperatur während 1 bis 250 Stunden, bis die abgekühlte Mischung eine Temperatur zwischen -20 °C und 30 °C erreicht hat, wodurch man eine abgekühlte Mischung erhält
f. Filtration der abgekühlten Mischung, wodurch man ein Filtrat erhält
g. Konzentration des Filtrats durch Destillation, wodurch man ein raffiniertes Extrakt erhält, das weniger als 15 % Massenanteil an Asiaticosid gegenüber der Gesamtmasse des Extrakts enthält

2. Verfahren nach Anspruch 1, wobei die Lösungsmittelmischung in Schritt a) eine Mischung von zumindest zwei Lösungsmitteln ist, die zumindest 10 % Massenanteil Wasser und ein alkoholisches Lösungsmittel enthält

3. Verfahren nach Anspruch 1 oder 2, wobei die Mazeration der überirdischen Teile der *Centella asiatica* bei einer Temperatur zwischen 50 °C und 70 °C erfolgt

4. Verfahren nach einem der Anspruche 1 bis 3, wobei die Mazeration der überirdischen Teile der *Centella asiastica* bei einer Temperatur um die 50 °C erfolgt

5. Verfahren nach einem der Ansprüche 1 bis 4, wobei die Filtration der Mazerationsmischung durch aufeinanderfolgende Filtervorgänge mit Filtern mit einer Filterfeinheit von 600 *µ*m, 100 *µ*m und anschließend 25 *µ*m erfolgt

6. Verfahren nach einem der Ansprüche 1 bis 5, wobei Schritt α) die folgenden Schritte vorausgehen:
⇒ Konzentration des erhaltenden Filtrats am Ende von Schritt a) und anschließend
⇒ Basifizierung mittels einer starken mineralischen Base vom Typ Alkalihydroxid (Lithiumhydroxid LiOH, Natriumhydroxid NaOH oder Kaliumhydroxid KOH) in Form eines Feststoffs oder einer wässrigen Lösung oder mit Branntkalk CaO oder auch mit Löschkalk Ca(OH)₂, und anschließend
=> Azidifizierung mit einer starken mineralischen Säure vom Typ Phosphorsäure H₃PO₄, Phosphorsäure HCI, Salpetersäure HNO₃, Schwefelsäure H₂SO₄, Perchlorsäure HCIO₄, Bromwasserstoffsäure Hbr oder auch mit einer organischen Säure
⇒ Filtration der azidifizierten Mischung

7. Verfahren nach einem der Ansprüche 1 bis 6, **dadurch gekennzeichnet, dass** das Adsorberharz von Schritt α vom Typ Styren-Divinylbenzen oder vom Typ Formophenol ist

8. Verfahren nach einem der Ansprüche 1 bis 7, **dadurch gekennzeichnet, dass** der Eluent in Schritt β 60 bis 70 % Massenanteil eines alkoholischen Lösungsmittels und genauer gesagt 60 bis 70 % Ethanol, sowie Wasser enthält

9. Verfahren nach einem der Ansprüche 1 bis 8, wobei Schritt b) die folgenden Schritte vorausgehen:
- Konzentration des erhaltenden Eluats durch Destillation des oder der alkoholischen Lösungsmittel
- Filtration des destillierten Eluats

10. Verfahren nach einem der Ansprüche 1 bits 9, wobei Schritt d) ein Schritt zur Trocknung unter Vakuum oder durch Zerstäubung vorausgeht

11. Verfahren nach einem der Ansprüche 1 bis 10, **dadurch gekennzeichnet, dass** die in Schritt d) hinzugefügte Lösungsmittelmischung 70 % Wasser und 30 % Methanol beinhaltet und auf eine Temperatur um die 60 °C gebracht wird

12. Verfahren nach einem der Ansprüche 1 bis 11, **dadurch gekennzeichnet, dass** die Absenkung der Temperatur in Schritt e) über einen Zeitraum von 100 bis 250 Stunden erfolgt, bis die Temperatur der abgekühlten Mischung zwischen -5 °C und 5 °C liegt

13. Verfahren für die Zubereitung eines gereinigten Extrakts aus *Centella asiatica* mit über 90 % Massenanteil einer Mischung aus Madecassoside und Terminoloside gegenüber der Gesamtmasse des Extrakts, wobei das Verfahren einen Schritt zur Reinigung des raffinierten Extrakts, das nach dem Verfahren gemäß einem der Ansprüche 1 bis 12 hergestellt wurde, umfasst und wobei der Reinigungsschritt die folgenden Schritte umfasst:
h. Hinzufügen einer Lösungsmittelmischung zum raffinierten Extrakt, das nach dem Verfahren gemäß einem der Ansprüche 1 bis 12 hergestellt wurde, wobei die Lösungsmittelmischung zumindest 85 % Masseanteil eines alkoholischen Lösungsmittels und gegebenenfalls Wasser enthält und auf eine Temperatur zwischen 30 °C und 50 °C gebracht wird, sodass man eine Lösung erhält
i. Langsames und schrittweises Absenken der Temperatur während 1 bis 250 Stunden auf eine Temperatur zwischen -15 °C und 15 °C, wodurch man eine abgekühlte Mischung erhält
j. Filtration der abgekühlten Mischung, wodurch man ein Filtrat erhält
k. Konzentration durch Destillation des Filtrats, wodurch man ein gereinigtes Extrakt erhält, das weniger als 6 % oder sogar weniger als 1 % Masseanteil an Asiaticosid gegenüber der Gesamtmasse des Extrakts enthält

14. Verfahren nach Anspruch 13, **dadurch gekennzeichnet, dass** die Lösungsmittelmischung in Schritt h) auf eine Temperatur um die 45 °C gebracht wird

15. Verfahren nach Anspruch 13 oder 14, **dadurch gekennzeichnet, dass** das Absenken der Temperatur in Schritt i) bis auf eine Temperatur der abgekühlten Mischung von um die -5 °C über einen Zeitraum von 100 bis 250 Stunden erfolgt

## Claims

1. Method for preparing a refined extract of *Centella asiatica* comprising more than 50 wt.% of a mixture of madecassoside and of terminoloside relative to the total weight of the extract, said method comprising the following steps:
a. macerating aerial parts or leaves of the *Centella asiatica* plant under stirring in water or in a solvent mixture comprising at least water and an alcohol solvent, at a temperature of 20°C to 90°C, for 20 to 180 minutes, in a [*Centella asiatica*]:[ solvent mixture] ratio between 1:2 and 1:20; then:
filtering the maceration mixture, whereby an aqueous solution is obtained;
α. passing the obtained aqueous solution over an absorbent resin capable of absorbing the triterpene heterosides;
β. eluting said absorbent resin with an eluent comprising at least 30 wt.% of an alcohol solvent and possibly of water, whereby an eluate is obtained; then:
treating the obtained eluate with active carbon;
b. passing the treated eluate over a discolouring resin of the strong anionic styrene-chloride type, then treating by successive passage(s) over a strong cationic ion exchanging resin comprising functional groups of the sulphonate type, then over a strong anionic ion resin comprising functional groups of the quaternary ammonium type or passing the eluate over said mixed resins, whereby a discoloured aqueous phase is obtained;
c. vacuum-concentrating the discoloured aqueous phase using tangential filtration membrane techniques any organic or mineral membrane by using media having a cut-off threshold between 200 Da and 100 kDA, whereby an extract is obtained; then:
recrystallising the obtained extract by:
d. solubising the obtained extract in a solvent mixture comprising water and an alcohol solvent brought to a temperature of 40°C to 70°C, whereby a solution is obtained;
e. slowly and gradually reducing the temperature for 1 to 250 hours to a cooled mixture temperature between -20°C and 30°C, whereby a cooled mixture is obtained;
f. filtering the cooled mixture, whereby a filtrate is obtained;
g. concentrating the filtrate by distillation, whereby a refined extract is obtained comprising less than 15 wt.% of asiatocisode relative to the total weight of the extract.

2. Method according to claim 1, wherein the solvent mixture in step a) is a mixture of at least two solvents comprising at least 10 wt.% of water and an alcohol solvent.

3. Method according to claim 1 or 2, wherein macerating aerial parts of *Centella asiatica* is carried out at a temperature between 50°C and 70°C.

4. Method according to any one of claims 1 to 3, wherein macerating aerial parts of *Centella asiatica* is carried out at a temperature of approximately 50°C.

5. Method according to any one of claims 1 to 4, wherein filtering the maceration mixture is carried out by successive filtrations using filters with porosity of 600 µm, 100 µm, then 25 µm.

6. Method according to any one of claims 1 to 5, wherein step α) is preceded by the following steps:
⇒ concentrating the filtrate obtained on completion of step a); then
⇒ basifying with a strong mineral base of the alkali hydroxide type (lithium hydroxide LiOH, sodium hydroxide NaOH or potassium hydroxide KOH) in the form of a solid or an aqueous solution or of quick lime CaO or even of slaked lime Ca(OH)₂; then
⇒ acidifying with a strong mineral acid of the phosphoric acid H₃PO₄, phosphoric acid HCI, nitric acid HNO₃, sulphuric acid H₂S0₄, perchloric acid HCIO₄, hydrobromic acid Hbr or even organic acid type; then
⇒ filtering the acidified mixture.

7. Method according to any one of claims 1 to 6, **characterised in that** the absorbent resin of step α is of the styrene-divinylbenzene type or of the formo-phenolic type.

8. Method according to any one of claims 1 to 7, **characterised in that** the eluent in step β comprises 60 to 70 wt.% of an alcohol solvent, and more specifically 60 to 70 % ethanol, and water.

9. Method according to any one of claims 1 to 8, wherein step b) is preceded by the following steps:
- concentrating the obtained eluate by distilling the one or more alcohol solvents;
- filtering the distilled eluate.

10. Method according to any one of claims 1 to 9, wherein step d) is preceded by a step of vacuum or atomisation drying.

11. Method according to any one of claims 1 to 10, **characterised in that** the solvent mixture added during step d) comprises 70 % water and 30 % methanol and is brought to a temperature of approximately 60°C.

12. Method according to any one of claims 1 to 11, **characterised in that** reducing the temperature during step e) is carried out over a period of 100 to 250 hours, to a cooled mixture temperature between -5°C and 5°C.

13. Method for preparing a purified extract of *Centella asiatica* comprising more than 90 wt.% of a mixture of madecassoside and of terminoloside. relative to the total weight of the extract, said method comprising a step of purifying the refined extract obtained according to the method according to any one of claims 1 to 12, said purification step comprising the following steps:
h. adding a solvent mixture to the refined extract obtained according to the method according to any one of claims 1 to 12 comprising at least 85 wt.% of an alcohol solvent and possibly of water brought to a temperature between 30°C and 50°C, whereby a solution is obtained;
i. slowly and gradually reducing the temperature of the solution for 1 to 250 hours to a temperature between -15°C and 15°C, whereby a cooled mixture is obtained;
j. filtering the cooled mixture, whereby a filtrate is obtained;
k. concentrating the filtrate by distillation, whereby a purified extract is obtained comprising less than 6 wt.%, and even less than 1 wt.%, of asiatocisode relative to the total weight of the extract.

14. Method according to claim 13, **characterised in that** the solvent mixture in step h) is brought to a temperature of approximately 45°C.

15. Method according to claim 13 or 14, **characterised in that** the temperature reduction in step i) is carried out over a period of 100 to 250 hours to a cooled mixture temperature or approximately -5°C.
